# EUROPEAN PATENT SPECIFICATION

(11) **EP 1 186 654 B1**
(45) Date of publication and mention of the grant of the patent: **28.07.2004**
(21) Application number: 00928009.0
(22) Date of filing: 18.05.2000
(51) Int. Cl.: C12N 1/20, A61K 35/74, A23C 9/12

(54) **BACTERIA STRAIN LACTOBACILLUS ACIDOPHILUS N.V. EP 317/402 NARINE AAA USED IN THE PRODUCTION OF PREPARATIONS AND OF DIETETIC, THERAPEUTIC AND PROPHYLACTIC PRODUCTS FOR TREATING DYSBACTERIOSIS AND THE CONSEQUENCES THEREOF**
BAKTERIENSTAMM LACTOBACILLUS ACIDOPHILUS N.V. EP 317/402 NARINE, WELCHER ZUR HERSTELLUNG VON ZUSAMMENSETZUNGEN UND VON DIäTETISCHEN, THERAPEUTISCHEN UND PROPHYLAKTISCHEN PRODUKTEN ZUR BEHANDLUNG VON DYSBAKTERIOSE UND DEREN FOLGEN VERWENDET WIRD
SOUCHE DE BACTERIE LACTOBACILLUS ACIDOPHILUS N.V. EP 317/402 NARINE AAA UTILISEE DANS LA PRODUCTION DE PREPARATIONS AINSI QUE DE PRODUITS DIETETIQUES, THERAPEUTIQUES ET PROPHYLACTIQUES PERMETTANT DE TRAITER LA DYSBACTERIOSE ET SES CONSEQUENCES

(30) Priority: 18.05.1999 RU 99109521
(43) Date of publication of application: 13.03.2002
(73) Proprietor: Anisimova, Taisiya Ivanovna, Novosibirsk, 630099 (RU)
(72) Inventor: ANISIMOVA, Taisiya Ivanovna, Novosibirsk, 630099 (RU); ARAKELYAN, Raisa Aramovna, Erevan, 375037 (AM)
(74) Representative: Kietzmann, Manfred
(86) International application number: PCT/RU2000/000183
(87) International publication number: WO 2000/070020

(56) References cited:
- EP-A- 0 199 535
- EP-A1- 0 465 677
- RU-C1- 2 001 578
- RU-C1- 2 035 871
- RU-C1- 2 103 353
- SALMINEN E ET AL: "PRESERVATION OF INTESTINAL INTEGRITY DURING RADIOTHERAPY USING LIVELACTOBACILLUS ACIDOPHILUS CULTURES" CLINICAL RADIOLOGY, LIVINGSTONE, HARLOW,, GB, vol. 39, no. 4, 1988, pages 435-437, XP000971553 ISSN: 0009-9260
- SHALEV E ET AL: "INGESTION OF YOGURT CONTAINING LACTOBACILLUS ACIDOPHILUS COMPARED WITH PASTEURIZED YOGURT AS PROPHYLAXIS FOR RECURRENT CANDIDAL VAGINITIS AND BACTERIAL VAGINOSIS" ARCHIVES OF FAMILY MEDICINE, AMERICAN MEDICAL ASSOCIATION, CHICAGO, IL, US, vol. 5, no. 10, 1996, pages 593-596, XP000915496 ISSN: 1063-3987
- WAGNER R D ET AL: "BIOTHERAPEUTIC EFFECTS OF PROBIOTIC BACTERIA ON CANDIDIASIS IN IMMUNODEFICIENT MICE" INFECTION AND IMMUNITY, AMERICAN SOCIETY FOR MICROBIOLOGY. WASHINGTON, US, vol. 65, no. 10, 1 October 1997 (1997-10-01), pages 4165-4172, XP002086394 ISSN: 0019-9567
- KAILASAPATHY K. AND CHIN J.: "Survival and therapeutic potential of probiotic organisms with reference to Lactobacillus acidophilus and Bifidobacterium spp." IMMUNOLOGY AND CELL BIOLOGY, AUSTRALASIAN SOCIETY OF IMMUNOLOGY, vol. 78, no. 1, February 2000 (2000-02), pages 80-88, XP002225111
- SHENDEROV B.A., "Meditsinskaya mikrobnaya ekologiya i funktsional - noe pitanie", Mikroflora cheloreka i zhivotnykh I ee funktsii, Volume 1, M. Grant 1998, pages 133-135

## Description

### Field of Invention

The invention relates to biotechnology and particularly relates to a *Lactobacillus* strain which has the ability to produce the interferon in monocellular human cells, to affect physiological and immunological processes in organism. The strain can be used for preparing preparations and dietary foodstuffs, including sour milk produce, directed to prophylactics and treatment of dysbacteriosis and consequences thereof.

### Background of Invention

Promising strains of this *Lactobacillus* species are known, which are used in creating of various preparations and sour milk produce, and promote the regulation of the intestinal microbiocenose and non-specific immune stimulation during intestinal dysbacterioses of various etiology. (Korshunov V. M. "Problemy regulatsii mikroflory kischechnika". *Journal of Microbiology, Epidemiology and Immunology* (in Russian), Moscow 1995, *3*, 48-52.)

The *Lactobacillus acidophilus* VKM V-2020 strain is known which is D-antibiotics-resistant antagonist against intestinal infection pathogens. This strain is recommended as a basis in developing antagonistic biological media for prophylaxis and treatment of intestinal diseases caused by the pathogenic and provisionally-pathogenic microflora. It may be used in developing bacterial preparations for the correction of microfunctional disorders of the gastrointestinal tract. (RU, 2063436 C1, Int. Cl. C 12 N 1/20, 08.02.94).

The *Lactobacillus acidophilus*-13 (Agaratz) strain is known, which affects positively the regenerative processes in the intestine in treating the gastrointestinal diseases. This strain is used to correct dysbacterioses of various type and character of newborn and adult patients.

The strain is used in manufacturing a therapeutic-dietary foodstuff promoting to inhibit a pathogenic microorganism growth and normalize an intestinal micriflora, because of the high maximum acidity of this strain of about 400°T whereby °T means the amount of ml of a 0,1 normal NaOH or KOH solution which is necessary to neutralize 100 ml or 100 g sour milk product (RU 2103354 C1, Int. Cl. C 12 N 1/20, 27.01.98).

The *Lactobacillus acidophilus*-92 n.v. (Ani) strain is known, which is used to correct dysbacteriosis during gastrointestinal diseases and for patients with the cancer of the large intestine (RU 2103353 C1 Int. Cl. C 12 N 1/20, 27.01.98).

Known *Lactobacillus acidophilus* n.v.317/402 "Narine" INMI 9602 strain possesses a slow acidogenic ability. Maximum formation is 360°T, whereby °T means the amount of ml of a 0,1 normal NaOH or KOH solution which is necessary to neutralize 100 ml or 100 g sour milk product. The strain produces a significant amount of antibiotic agents non-harmful for children, which inhibit a growth and development of both gram-positive and gram-negative bacteria causing acute forms of gastrointestinal diseases (SU 163357 A, Int. Cl. C 12 N 1/20, 1964).

Long-term observations reveal the absence of phagolysis in the *L. acidophilus* n.v. 317/402 "Narine" strain. An unskimmed milk product is prepared by a fermentation of unskimmed milk at 28-40°C with 1 mass-% of the strain. After 5-8 hours the product curdles, the product acidity being 60-90°T, whereby °T means the amount of ml of a 0,1 normal NaOH or KOH solution which is necessary to neutralize 100 ml or 100 g sour milk product. Said product is suitable as a leaven as well as a therapeutic/prophylactic means in diseases of the gastrointestinal tract. The product is used in a medical and veterinary practice.

A product obtained using the *L. acidophilus* n.v. 317/402 "Narine" strain with acidity not greater than 90°Twhereby °T means the amount of ml of a 0,1 normal NaOH or KOH solution which is necessary to neutralize 100 ml or 100 g sour milk product, does not provide a human being with the whole complex of amino acids and vitamins required to correct the dysbacteriosis and various gastrointestinal diseases.

### Disclosure of the Invention

The causes of dysbacteriosis are well known: they include various infectious diseases, antibiotics administration, chemical, hormonal and radiation therapy, psycho-emotional stresses, unfavourable ecological situation, social factors, alcoholism, and so on. These factors promote, directly or indirectly, an microbiocenose disbalance of an organism, and first of all of the intestinal microflora.

In order to treat the dysbacteriosis it is necessary, if possible, to eliminate its cause and to recover the microflora.

Thus, the actual problem is to disclose the novel lactobacillus strains and to develop, on the basis of these strains, preparations and foodstuffs for the prophylactics and recovery of the normal function of the intestinal microflora, and for elimination of dysbacteriosis and consequences thereof.

It is an object of the present invention to provide the novel *L. acidophilus* n.v. EP 317/402 "Narine" AAA strain which complies with the modern requirements regarding preparations and therapeutic/prophylactic produce, possesses a resistance against a series of antimicrobial preparations, exhibits a high acidogenic activity and explicit antagonistic properties in regard to provisional-pathogenic and pathogenic microorganisms; possesses a high vitamin-producing ability, heightened α- and γ-interferon productivity in monocellular human cells as crucial factors in antiviral and anticancer defence, and affects the physiological processes in an organism.

The *L. acidophilus* n.v. EP 317/402 "Narine" AAA strain has been obtained by a long-term selection of the original *L. acidophilus* n.v. 317/402 "Narine" strain culture and directed selection according to the following indicators: an antibiotical activity against gastrointestinal disease pathogens; an antibiotical resistance; reproductive dynamics of the bacterial population in a culture medium; a vitamin-producing activity; and the amount of viable bacteria in 1 ml of the strain constituting about 220-250 millions of the lactobacillus strain cells.

The comparative data regarding the antagonistic activity of the proposed and known strains are summarized below in the Table 1.

**Table 1**

| Test culture | Growth inhibition zone for the test culture around *L. acidophilus* colonies, mm | |
|---|---|---|
| | *L. acidophilus* n.v. 317/402 "Narine" | *L. acidophilus* n.v. 317/402 "Narine" AAA |
| *Staphylococcus aureus* | 30-35 | 32-28 |
| *Escherichia coli* | 24-28 | 26-30 |
| *Salmonella typhimurium* | 24-26 | 24-27 |
| *Klebsiella* sp. | 23-26 | 26-28 |
| *Pseudomonas aeroginosa* | 25-29 | 27-32 |
| *Serratia marcescens* | 26-28 | 27-30 |
| *Bacillus subtilis* | 25-27 | 27-29 |

The filed strain exhibits an heightened antagonism against the above-mentioned representatives of the enteropathogenic intestinal microflora.

It has been established that the *L. acidophilus* n.v. 317/402 "Narine" AAA is not sensitive to the sulphamethoxine, ethazole, sulphadene, sulphatone, sulphadimesine, biseptol, norsulphazole, tarivid, nalide, ampioxe, negram and gramurine.

Moreover, it has been found that the strain has an heightened amount of aromatic compounds - up to 6.9 mg %, amino acids - up to 23 mg %, ascorbic acid - up to 1.20 mg %, riboflavine - up to 1680 mg %, whereby mg % means the weight content of the component(s) in 100 g sour milk produkt.

The strain also exhibits cellulolytic activity. It has a higher growth rate than the origin strain, which is the positive factor for manufacturing.

The *L. acidophilus* n.v. 317/402 "Narine" AAA strain is deposited in the All-Russian Collection of Industrial Microorganisms (ARCIM) V-7747 and in the Collection of Microorganism Cultures of SSC "Vektor" (No. V-741) as intended for preparing of the dry tablet-shaped "Narine" preparation.

### Culture-morphological and biochemical properties of the strain

Homoenzymatic immobile gram-positive microaerophilic bacilli of the size of 2 to 200 µm exhibit a high resistance to phenol - up to 0.4-0.5%; phtalozoles content - up to 1%; synthomicine stability - up to 0.03%; they are able to produce a significant amount of harmless antibiotic substances which inhibit the growth and development of both gram-positive and gram-negative bacteria (E. coli, all dysentery-inducing bacteria etc.).

The strain neither reduces nitrates, nor generates indol, nor dilutes gelatin, nor possesses hemolytic activity. It ferments lactose, glucose, sucrose, fructose, mannose, levulose, galactose, maltose, rafinose, starch, dextrin, sorbit, dulcite without forming a gas, during which fermentation basically the lactic acid and a small amount of volatile acids are formed, said amount does not exceed 5-6% of the total amount of the titrated acids. The strain also ferments milk in 4-5 hours at 37°C. It does not produce the catalase. The strain is homoenzymatic and has the ability to form flavoproteinic proteases. It synthesizes vitamines of B and C groups, folic acid, thiamine, riboflavin, vitamines E and PP, and nicotinic acid.

The strain possesses a slow acidogenic ability with the minimum activity of 90°T, the optimal activity up to 120-160°T and the maximum activity up to 380°Twhereby °T means the amount of ml of a 0,1 normal NaOH or KOH solution which is necessary to neutralize 100 ml or 100 g sour milk product. The amount of viable bacteries in 1 ml of the strain is 150-250 millions of living lactobacillus cells.

### Stability against antibiotics

The strain has a low sensitivity in regard to: hentamycine, oletetrine, tetracycline, kefzole, rifampicin, neomycine, canamycine, amicacine, oleandromycine, nitroxolone, furadonine, cetazole, metracidozole, fusidine and klaforane.

The strains has no sensitivity in regard to: sulphamethoxine, ethazole, sulphadene, sulphitone, sulphadimezine, norsulphazole, biseptol, tarivid, naline, ampioxe, negram, gramurine.

The strain stability against antibiotics allows to use it in a bacterial and antibiotical therapy with increasing sanitating and prophylactic effect.

Assays of the "Narine" preparation performed with volunteers and diseased patients have approved the high immunomodulating activity of the preparation. The dynamics of functional organism condition was studied for 1 hour after administering the dry lyophilised *L. acidophilus* n.v. EP 317/402 "Narine" AAA strain preparation. The preparation was administered every morning for 30 days, one packet (0.3 g) at 30 minutes before meal. Organism responses were registered by finger irradiation before administering of the preparation, and then taking photos every 10 minutes for one hour. Analysis of the bioelecrograms has shown that the administration of the preparation resulted, for patients with the normal bioenergetic status, in improvement of the luminescence pattern, which was maintained during the whole observation time.

The estimation of the functional condition of organs and systems during this hour-long monitoring has revealed the dynamics of conditions in the sectors of projections of the large and small intestines, peripheral and encephalic blood circulation, endocrinal system and kidneys. All patients showed the best results 40 minutes after the administration of the "Narine" preparation, then (after 1 hour) these results were slightly reduced though still being 0.5-1.0 points higher than the initial values.

The obtained results show objectively that the correcting effect of the "Narine" preparation manifests as soon as 10 minutes after the administration, when the assimilation is not yet present.

This fact can be explained with the energy-informational effect which of field components of living lactobacilla onto the human energy-informational system as well as by correcting effect of the "Narine" preparation lactobacilla, particularly in recovering the human organism microbiocenose.

The noted positive dynamics of a bioenergetic and psychoemotional status, functional conditions of the main organs and systems at the end of the preparation administering course allows to recommend the "Narine" preparation for the background therapy at multiple diseases.

The course of dysbacteriosis correction in patients using the sour milk dietary "Narine" beverage has not only reduced symptoms of a gastrointestinal disease but also revealed a significant improvement in the cardiovascular activity including sections of both central, humoral, and vegetative regulation. The latter are related to the reduction of the organism intoxication with vital activity products of pathogenic bacterial microflora.

In the local hepatic lymph node the "Narine" preparation results in structural changes following with the reduction of corticocerebral index down to 0.54-0.76 (0.45 in reference). The cerebral substance prevails over the cortical substance in the lymph node. In the cortical substance the plateau positively reduces 1,6 times. The area of primary lymph nodes is 2,5 times greater than the area of secondary ones. There has been noted a positively reduction of the paracortex area down to 23.9 (33.8 in reference). In the cerebral substance the area of fiber cords increases 1.4 times and the area of cerebral sinus increases 1.5 times. In the terminal centres the quantitative density of lymphoplasts and macrophages increases. The tendency takes place to increase the mitotic activity and to reduce degeneration processes. The total quantitative density of lymphocyte cells increases significantly, and that of small lymphocytes increases 1.2 times. The total quantitative density of medium lymphocytes increases 1.5 times. The quantitative density of macrophages decreases 1.3 times.

Thus, administering the "Narine" preparation results in intensifying the drainage function on the background of the humoral immunity section activity, taking into account the character of morphologic changes of structural-functional zones of the hepatic lymph node.

Application of the "Narine" preparation in the case of the toxic hepatitis stabilizes the ratio of cortical and cerebral substance in the lymph node on the level of reference values, said ratio being equal 0.6-0.78 (compared to 1.07-1.48 in the case of the toxic hepatitis). The corticocerebral index value indicates a prevailing part of the cerebral substance in the structure of the lymph node. In the cortical plateau the area occupied with primary and secondary lymphoid knots remains positively constant. The paracortex area does not expand and remains at the reference level values of 23.31-25.89, which is 1.5 times less than the area in the case of the hepatitis (32.58-42.36 for the toxic hepatitis) in reference (21.64-30.84). The area of fiber cords in the cerebral substance increases 1.4 times. The cerebral sinus value changes not surely.

Lymphoblasts amount in the terminal centres doubles while small lymphocytes amount reduces 1.4 times. It is detected 20% reduction of medium lymphocytes and 40% reduction of small lymphocytes in the sinuses. The tendency appears to increase the amount of matured and non-matured plasmocytes and to double the amount of lymphoblasts.

Thus, on the level of the mesenteric lymph node it is observed the enchancement of drainage function along with the activation of the humoral section and stabilization of the structures responsible for the cell immunity.

The use of the "Narine" preparation produced using the *L. acidophilus* n.v. EP 317/401 "Narine" AAA strain for correcting the hepatitis-caused dysbacteriosis has resulted in normalization of the intestinal microflora, reduction of the toxic pressure, but has not altered the pathological process development within the liver.

The lymphatic system condition while administering the "Narine" eubiotic preparation has been examined on white rats in order to determine lymphotrophic properties of the preparation. The obtained results show that the "Narine" preparation normalizes the gastrointestinal ecosystem, thereby exhibiting lymphotrophic properties with respect to the lymphatic system.

Application of the "Narine" product in the case of the endotoxicosis stabilizes the structural response of lymph nodes and does not increase the toxic pressure due to the recovery of the microbiocenose.

Of particular importance is the fact of determining that the *L. acidophilus* n.v. EP 317/401 "Narine" AAA strain exhibits a relatively high cellulosic activity. A periodic application of the "Narine" preparation produced on the basis of the filed strain as a food additive prevents undesirable consequences concerned with the lack of natural cellulase microbes in the intestines.

The use of the "Narine" preparation containing not less than 5×10⁸ CFU/g in 500-700 ml doses daily stipulates a creation of optimal conditions for natural endogenic flora growth recovery.

An administration of the "Narine" preparation as a part of the complex therapy to the group of dysentery patients consisted of 428 persons of 15 to 55 years old has showed a termination of the diarrhea syndrome, intoxication, reduced the reconvalescency time, and reduced a dysbacteriosis development frequency for 58% compared with the reference group.

These data were approved by *in vivo* assays accomplished on gnotobionts of the human nature. A study of the adhesion kinetics of the *E. coli* to the intestine epithelium of a gnotobiont has showed that in a joint employment of cultures of the *E. coli* and *L. acidophilus* n.v. EP 317/401 "Narine" AAA lactobacillus strain the adhesive ability of the *E. coli* increases significantly.

The above described effect is most expressed in the case of the high lactobacillus concentration which, in clinical practice, implies the need of usage of eubiotics having a high content of microbial cells per 1 ml or a gram of a dry matter.

The obtained data make ground to recommend widely the "Narine" preparation produced using the filed strain as an eubiotic for a treatment of acute intestinal infections.

In a group of children of 7 to 14 years old suffering from allergic rinites, observed in the Byelokourikha children health centre, were detected: the nasal respiration instability, nasal obstruction, abundant nasal mucus secretion, nasal itch, eosinophilia in clinical blood tests.

The main clinical symptoms in the group of children suffering from frequent acute respiratory diseases were: nasopharingal catarrhal manifestations, stagnation phenomena in pharyngal mucosa, headaches, functional disorders of the central nervous system.

The main symptoms in the group of children suffering from neurodermatites were skin changes, itch intensified at night, reduced appetite, irascibility, irritability, bad sleep, changes in clinical blood tests (eosinophilia).

As a result of the application of the "Narine" preparation for therapeutic treatment of this children group, all children have exhibited the positive dynamics in the most part of clinical manifestations registered at the moment of entering the health centre.

Thus, in the group of children suffering from neurodermatites, the skin itch of 37 children (88%) was reduced, the recovery of the epidermal layer of 28 children (66,7%) was observed, the epidermal layer colour of 36 children (85,7%) was improved, the normalization of coprogram values was observed, the stool of 50 children (94,3%) was normalized, and 48 children (90,6%) exhibit an increasing appetite.

In the group of children suffering from allergic rinites, the respiration of 23 children (92%) was recovered, the eosinophilia of 20 children (80%) was reduced, and the pharyngal mucosa condition of 21 children (84%) was improved.

The average amount of procedures per child was 28. The product was administered in courses of 10 to 20 days. the 10-days course was administered to 27 children, the 14-days course was administered to 67 children, and 20-days course was administered to 80 children. The reference group consisted of 30 children receives a complex sanatorium treatment (balneotherapy, phytotherapy, massage, therapeutic physical training) without the "Narine" preparation use. It was determined that, in the reference group, the health conditions of 90% of children suffering from neurodermatites, dysbacteriosis, chronical gastroduodenitis were improved. In the group where the "Narine" preparation was used, 98,7% of patients showed significant improvements.

Thus, the results of the accomplished assays shows the positive effect of the course of administering the "Narine" preparation onto the clinical manifestation dynamics for children suffering from various diseases, which allows to recommend the preparation for the complex sanatorium treatment.

Taking into account the actuality of the dysbacteriosis problem in the case of the children burn trauma, an assay was accomplished in Novosibirsk Interterritorial Burn Centre in order to study a dysbacteriosis development frequency of children having burns and receiving the antibacterial therapy, as well as the fect of the "Narine" product produced using the filed strain onto the recovery of the intestinal microbiocenose. The group consisted of 16 patients of 1 to 7 years old having burn area of 4 to 52%, including 4 to 29% for the deep burn. The reference group consisted of 12 patients of 1 to 6 years old having burn area of 5 to 40%, including 2 to 30% for the deep burn. In both groups the conventional methods of a conservative and operational treatment were used, but patients of the reference group did not receive the biopreparation. The "Narine" preparation was administered as an aqueous solution twice a day 1 flask (0,3 g) at 30 minutes before mealtime. The leukocytes amounts, leukocytic activity index and bacteriologic microfloral excremental tests were monitored in the beginning of the treatment and after healing the wounds.

The assay has showed that an application of the "Narine" preparation as a food additive promoted to normalize the blood leukocytes and to reduce the leukocytic activity index, which evidences the increase of the organism resistance and the desintoxicating effect. An increase of bifidobacteries concentration in excrement being a result of the application of the preparation shows the positive clinical result with the partial dysbacterioses correction. Moreover, two patients from the reference group exhibited a gastrointestinal dysfunction (vomiting, diarrhoea) before the treatment; after the treatment two other children showed similar manifestations. There were no such cases in the test group. Revealed properties of the "Narine" preparation allow to consider it as a lymphotrophic medicament. An administration of the "Narine" preparation affects a condition of structural-functional zones of lymph nodes, determining an activity of the humoral and cellular immunity.

The sour milk "Narine" preparation were tested as a veterinary preparation and was proved to recover effectively the intestinal microbiogenesis of calves and recommended as means for improving calves' conditions in the cattle-breeding. The *L. acidophilus* n.v. EP 317/402 "Narine" AAA strain can be used for treating the dysbacterioses and diseases related to disorders of the normal intestinal microflora both in humans and in animals.

### Preferred embodiment of the Invention

### Example 1.

In order to obtain the leaven a sterilized or pasteurized milk is fermented at 37-39°C with the *L. acidophilus* n.v. EP 317/402 "Narine" AAA culture at 2% of the total milk amount. The mixture is incubated for 28 hours. The fermented leaven is cooled to 6°C, and allowed to stay at this temperature for 8 hours. The obtained clot has the acidity of 160°Twhereby °T means the amount of ml of a 0,1 normal NaOH or KOH solution which is necessary to neutralize 100 ml or 100 g sour milk product, 1 ml of the liquid leaven comprises up to 250 millions of living cells. The obtained leaven can be used as a bacterial preparation having good selection properties for producing a dietary and therapeutic food stuffs or for a direct application in food. The product in the form of dry leaven is used as a means for the treatment and prophylactics of various diseases including secondary immunodeficiency.

### Example 2.

In order to prepare the sour milk "Narine" product, 2% of the leaven of *L. acidophilus* n.v. EP 317/402 "Narine" AAA strain is added to an unskimmed milk at 38-39°C. The mixture is stirred and allowed to stay at 30°C for 5 hours or for 6 hours at a room temperature until a clot having the acidity of 80-90°Twhereby °T means the amount of ml of a 0,1 normal NaOH or KOH solution which is necessary to neutralize 100 ml or 100 g sour milk product, is created, then the mixture is placed into a refrigerator for 2-3 hours. The quality of the produced beverage maintains for 1 week at 4-6°C.

A nourishing value of the "Narine" product corresponds to 820-870 cal. The product has the acidity of 130°Twhereby °T means the amount of ml of a 0,1 normal NaOH or KOH solution which is necessary to neutralize 100 ml or 100 g sour milk product, and comprises 0.610% of nitrogen and 24.7 mg % of amine nitrogen.

### Industrial applicability

The product obtained on the basis of the *L. acidophilus* n.v. EP 317/402 "Narine" AAA strain is industrially applicable, has been clinically tested in the Institute for Proctology of The Republic of Armenia and at the Gamaleya Institute for Epidemiology of The Russian Academy of Medical Sciences.

Clinical assays showed that the strain is able for the active settling in mucosa of the digestive tract and large intestine, as well as for maintaining a normal intestinal biocenose and correcting a dysbacteriosis in various gastrointestinal diseases.

## Claims

1. Lactobacillus acidophilus n.v. EP 317/402 "Narine" AAA strain deposited under deposition number VKPM V-7747 at All-Russian Collection of Industrial Microorganisms (ARCIM).

2. Use of the strain Lactobacillus acidophilus n.v. EP 317/402 "Narine" AAA deposited under deposition number VKPM V-7747 at All-Russian Collection of Industrial Microorganisms (ARCIM) for preparing preparations for prophylaxis and treatment of dysbacteriosis and consequences thereof.

3. Use of the strain Lactobacillus acidophilus n.v. EP 317/402 "Narine" AAA deposited under deposition number VKPM V-7747 at All-Russian Collection of Industrial Microorganisms (ARCIM) for preparing dietary foodstuffs.

## Patentansprüche

1. Stamm Lactobacillus acidophilus n.v. EP 317/402 "Narine" AAA, hinterlegt unter der Nummer VKPM V-7747 bei der Allrussischen Sammlung Industrieller Mikroorganismen (ARCIM).

2. Verwendung des Stamms Lactobacillus acidophilus n.v. EP 317/402 "Narine" AAA, hinterlegt unter der Nummer VKPM V-7747 bei der Allrussischen Sammlung Industrieller Mikroorganismen (ARCIM), für die Zubereitung von Präparaten für die Prophylaxe und Therapie von Dysbacteriose und ihrer Folgen.

3. Verwendung des Stamms Lactobacillus acidophilus n.v. EP 317/402 "Narine" AAA, hinterlegt unter der Nummer VKPM V-7747 bei der Allrussischen Sammlung Industrieller Mikroorganismen (ARCIM), für die Zubereitung von diätetischen Nahrungsmitteln.

## Revendications

1. Souche Lactobacillus acidophilus n.v. EP 317/402 « Narine » AAA déposée sous le numéro VKPM V-7747 à All-Russian Collection of Industrial Microorganisms (ARCIM).

2. Usage de la souche Lactobacillus acidophilus n.v. EP 317/402 « Narine » AAA déposée sous le numéro VKPM V-7747 à All-Russian Collection of Industrial Microorganisms (ARCIM) pour la fabrication de préparation pour la prophylaxie et le traitement du dysmicrobisme et de ses suites.

3. Usage de la souche Lactobacillus acidophilus n.v. EP 317/402 « Narine » AAA déposée sous le numéro VKPM V-7747 à All-Russian Collection of Industrial Microorganisms (ARCIM) pour la préparation d'aliments diététiques.
